# FASCICULE DE BREVET EUROPEEN

(11) **EP 0 354 115 B1**
(45) Date de publication et mention de la délivrance du brevet: **06.04.1994**
(21) Numéro de dépôt: 89402183.1
(22) Date de dépôt: 01.08.1989
(51) Int. Cl.: G01S 13/89, G01N 22/00, G01N 33/38

(54) **Dispositif d'émission et de réception d'un rayonnement micro-onde, pour imagerie d'objets enfouis**
Sende- und Empfangsvorrichtung für Mikrowellenstrahlung zur Abbildung von verdeckten Gegenständen
Microwave transceiver for displaying concealed objects

(30) Priorité: 03.08.1988 FR 8810479
(43) Date de publication de la demande: 07.02.1990
(73) Titulaire: CENTRE NATIONAL DE LA RECHERCHE SCIENTIFIQUE (CNRS), 75700 Paris Cedex 07 (FR); ETAT FRANCAIS - LABORATOIRE CENTRAL DES PONTS ET CHAUSSEES, 75732 Paris Cédex 15 (FR); SOCIETE D'APPLICATIONS TECHNOLOGIQUES DE L'IMAGERIE MICRO-ONDE, 91952 Les Ulis Cédex (FR)
(72) Inventeur: Pichot, Christian, F-78100 Saint-Germain-En-Laye (FR); Chommeloux, Luc, F-78000 Versailles (FR); Picard, Dominique, F-92220 Bagneux (FR); Bolomey, Jean-Charles, F-75005 Paris (FR)
(74) Mandataire: Bloch, Gérard

(56) Documents cités:
- EP-A- 0 069 628
- WO-A-88/03656
- FR-A- 2 241 884
- FR-A- 2 534 698
- MICROWAVE JOURNAL, février 1981, pages 45-53, Dedham, US; C. CHEKROUN et al.: "RADANT: New method of electronic scanning"

## Description

La présente invention a pour objet un dispositif d'émission et de réception d'un rayonnement, disposé dans un premier milieu en vue de la formation, depuis ce premier milieu, d'une image d'un objet noyé dans un deuxième milieu.

L'invention s'applique notamment au cas ou l'on cherche à obtenir, de façon non destructive, une image des barres et réseaux de barres métalliques noyés dans du béton, pour la surveillance, l'entretien, la réparation ou le renforcement d'ouvrages ou bâtiments en béton armé, dont il n'existe plus aucun plan, par exemple. Dans ce cas, il est important de disposer d'un appareil permettant de déterminer le diamètre, le nombre, et la position des barres dans le béton sans avoir à y pratiquer de fenêtres pour mettre localement à nu les armatures, ce qui est fastidieux, et parfois dangereux. Naturellement, l'invention peut s'appliquer à tout autre domaine dans lequel il est utile de pouvoir former,de façon non destructive, des images d'objets enfouis.

On connaît déjà des dispositifs du type défini ci-dessus qui mettent en oeuvre un champ magnétique basse fréquence, et sa perturbation par tout objet métallique disposé dans son voisinage. Ces dispositifs sont connus par l'homme du métier sous le nom de "pachomètres" ou de "profomètres". Ils peuvent donner des indications sur la position des armatures les plus proches des parois, et il est en principe possible de déterminer, à l'aide d'abaques, le diamètre d'une barre si l'on connait l'épaisseur du béton qui l'enrobe, et inversement. Toutefois, dans la pratique, la précision de ces dispositifs est telle qu'ils permettent de détecter la présence ou l'absence de barres métalliques, dans un certain volume, sans qu'il soit véritablement possible de connaître leur nombre, leurs profondeurs et leurs diamètres.

Il en est de même des dispositifs selon le préambule de la revendication 1, tels que décrit dans le document WO-A-8 803 656.

On connaît aussi des dispositifs basés sur l'utilisation de rayons γ, ou de rayons X, comme dans les systèmes de radiographie ou de radioscopie du domaine médical. Toutefois, ces dispositifs, qui mettent en oeuvre de puissantes sources pour produire les rayonnements, sont très lourds, et très coûteux à mettre en oeuvre, et nécessitent un personnel nombreux et qualifié. De plus, ils ne sont pas sans risque d'irradiation pour le personnel. Par ailleurs, sur les clichés obtenus, il est souvent impossible d'interpréter l'image dans les zones à fortes densité de feraillage, et délicat de déterminer avec assez de précision le diamètre de barres dont la position par rapport à la source est inconnue. Enfin, ces dispositifs fonctionnent en transmission, ce qui complique la mise en place, et pose certains problèmes si le volume à ausculter est épais.

La présente invention vise à pallier les inconvénients précédents en procurant un dispositif du type défini ci-dessus, permettant une détermination précise et rapide de la localisation et de la forme des objets à examiner, travaillant en réflexion pour une mise en place facile, tout en étant pratiquement sans danger pour le personnel opérateur, portable et d'un coût modéré.

A cet effet, la présente invention a pour objet un dispositif selon la revendication 1.

Dans le dispositif de l'invention, le rayonnement utilisé est un rayonnement micro-onde, émis par l'antenne d'émission. Ce rayonnement émis, lorsqu'il parvient sur la surface de séparation, est en partie transmis dans le deuxième milieu, par exemple du béton. Ce rayonnement transmis est en partie réfléchi vers la surface de séparation à chaque fois qu'il rencontre, dans le béton, une discontinuité de permittivité ou de conductivité, par exemple. Un objet enfoui dans le béton est évidemment à l'origine de telles discontinuités qui peuvent donc être détectées à partir de la connaissance du rayonnement réfléchi auxquels elles donnent naissance. L'antenne de réception collecte ce rayonnement réfléchi et permet, grâce aux antennes ponctuelles, la mesure, en une pluralité de points, du champ corregspondant, lié à la présence de l'objet enfoui, en vue de la formation d'une image de cet objet.

La résolution spatiale, notamment dans la direction perpendiculaire à la surface de séparation, d'une telle image, image représentative de la permittivité et de la conductivité du matériau, peut notamment être améliorée, à partir des résultats d'une série de mesures à des fréquences micro-onde différentes, en utilisant la méthode exposée dans l'article :
"Electromagnetic Modeling for Microwave Imaging of Cylindrical Buried Inhomogeneities" par L. CHOMMELOUX, C. PICHOT et J.C. BOLOMEY dans IEEE Transactions on Microwave Theory and Techniques, Vol. MTT-34, N° 10, October 1986.

Dans le dispositif de l'invention, le rayonnement micro-onde utilisé est pratiquement sans danger, et les composants micro-onde mis en oeuvre sont d'un poids compatible avec la mobilité du dispositif, et d'un coût modéré. Du fait que, dans le matériau considéré, la longueur d'onde des rayonnements micro-onde est relativement faible, la résolution obtenue est satisfaisante. De plus, le type et la disposition des antennes d'émission et de réception permettent des mesures en réflexion, donc ne nécessitant pas de mise en place fastidieuse, et néanmoins précises, simples, et rapides, du fait que le rayonnement collecté par l'antenne de réception représente principalement le rayonnement utile, c'est-à-dire celui qui provient des réflexions sur l'objet enfoui.

En effet, un problème auquel on se heurte lorsque l'on cherche à faire, comme ci-dessus, des mesures en réflexion, est la présence d'un rayonnement micro-onde parasite, qui provient de la reflexion du rayonnement émis par l'antenne d'émission sur la surface de séparation. Ce rayonnement est inutile à la mesure puisque, ne pénétrant pas dans le deuxième milieu, il n'est porteur d'aucune information utile. Cependant, il est dirigé dans la même direction que le rayonnement utile, et, en général, en cherchant à mesurer celui-ci, on mesure en fait la somme du rayonnement utile et du rayonnement parasite. On dit encore que l'on mesure la superposition de la réponse de la surface de séparation, ou interface, et de la réponse de l'objet. Dans un tel cas,pour séparer la réponse de l'objet de la réponse de l'interface, il est possible d'effectuer une mesure de référence, le dispositif étant disposé dans une zone de la surface de séparation choisie pour que le second milieu ne comporte aucun objet enfoui au voisinage de cette zone, afin de déterminer la réponse de l'interface. Ensuite, on retranche, du résultat de la mesure globale, le résultat de la mesure de référence, afin de connaître la réponse de l'objet. Une telle méthode est évidemment longue et complexe à mettre en oeuvre, et imprécise, du fait notamment des variations de la réponse de l'interface entre la mesure de référence et la mesure globale.

Dans le dispositif de l'invention, le problème ne se pose pas, car il n'est pas nécessaire d'effectuer de mesure de référence. En effet, du fait que l'antenne d'émission se termine par une ouverture rayonnante appliquée contre l'interface, la majeure partie du rayonnement réfléchi par l'interface reste confinée à l'intérieur de l'antenne d'émission, et seule une partie résiduelle de ce rayonnement parasite, peu gênante, se trouve collectée par l'antenne de réception qui, par contre, par sa forme et sa disposition, collecte efficacement le rayonnement relatif à la réponse de l'objet enfoui. Ainsi, dans le dispositif de l'invention, il n'est pas nécessaire d'effectuer de mesure de référence, ce qui procure un gain appréciable sur la durée, la simplicité et la précision de la mesure.

Avantageusement, une couche de matériau absorbant le rayonnement micro-onde est intercalée entre le grand côté de ladite ouverture rayonnante et le grand côté de la dite ouverture collectrice qui se trouvent côte à côte.

Dans ce cas, l'antenne d'émission et l'antenne de réception sont très bien découplées l'une de l'autre, et ne s'influencent pas. Notamment, la partie résiduelle de la réponse de l'interface qui se trouve collectée par l'antenne de réception est encore réduite, et la précision des mesures s'en trouve améliorée.

Avantageusement encore, lesdites antennes ponctuelles sont noyées dans une lame diélectrique agencée pour assurer l'adaptation de ladite antenne de réception audit deuxième milieu.

Dans ce cas, la partie du rayonnement utile qui se réfléchit sur la surface de séparation en passant du deuxième milieu, par exemple le béton, au premier milieu, par exemple l'air ambiant, est réduite, et la partie transmise vers l'antenne de réception augmentée, ce qui augmente le niveau du champ, donc la sensibilité. De plus, la lame d'adaptation permet de maintenir et de protéger mécaniquement lesdites antennes ponctuelles, qui sont par exemple des antennes du type doublet électrique, chacune étant chargée en son centre par une diode.

Dans la forme de réalisation préférée, il est prévu une unique antenne de réception et deux antennes d'émission disposées symétriquement par rapport à ladite antenne de réception.

Dans cette forme de réalisation, la symétrie de l'éclairement permet une meilleure détermination de la tranche de volume analysée, et donc une localisation plus précise du ou des objets enfouis.

Dans une autre forme de réalisation, il est prévu une pluralité d'antennes d'émission et une pluralité d'antennes de réception, alternées.

Dans ce cas, la tranche de volume analysée est très épaisse, et on peut obtenir une image de cette tranche épaisse sans déplacement du dispositif.

Avantageusement encore :
- lesdits moyens de détermination du champ comprennent des moyens de détection synchrone micro-onde dudit signal micro-onde collecté suivis de moyens de détection et de traitement en basse fréquence,
- ladite source micro-onde, ladite antenne d émission, ladite antenne de réception et lesdits moyens de détection synchrone micro-onde sont tous solidaires d'un premier châssis portable pour être déplacé en divers endroits de ladite surface de séparation, et,
- lesdits moyens de modulation et lesdits moyens de détection et de traitement en basse fréquence sont reliés audit premier châssis par au moins un câble souple.

Ainsi, le déplacement des antennes pour effectuer des mesures en différenteszones de la surface de séparation n'apporte aucune perturbation dans les circuits micro-onde .

Avantageusement encore, lesdites antennes d'émission et de réception sont des antennes large bande.

Dans ce cas, la méthode citée d'amélioration de la résolution spatiale de l'image à partir d'une série de mesures effectuées à des fréquences micro-onde différentes peut être mise en oeuvre par un logiciel chargé sur une machine disposée en aval du dispositif, l'ensemble étant facilement agencé pour que la série de mesures et son traitement soit effectués automatiquement.

La présente invention sera mieux comprise grâce à la description suivante de plusieurs formes de réalisation du dispositif de l'invention, faite en se référant aux dessins annexés, sur lesquels :
- la figure 1 représente une vue en perspective du dispositif de l'invention,
- la figure 2 représente une vue de dessous de la tête de mesure du dispositif de la figure 1,
- la figure 3 représente une vue latérale, en coupe partielle, de la tête de mesure de la figure 2,
- la figure 4 représente un schéma par blocs du circuit micro-onde de la tête de mesure du dispositif de la figure 1,
- la figure 5 représente un schéma par blocs du circuit basse-fréquence du dispositif de la figure 1,
- la figure 6 représente une vue latérale, en coupe partielle, d'une première variante de la tête de mesure de la figure 2,
- la figure 7 représente une vue latérale d'une deuxième variante de la tête de mesure de la figure 2,
- la figure 8 représente une vue en perspective d'une variante de réalisation des antennes du dispositif de la figure 1, et,
- la figure 9 représente une vue de dessous des antennes de la figure 8.

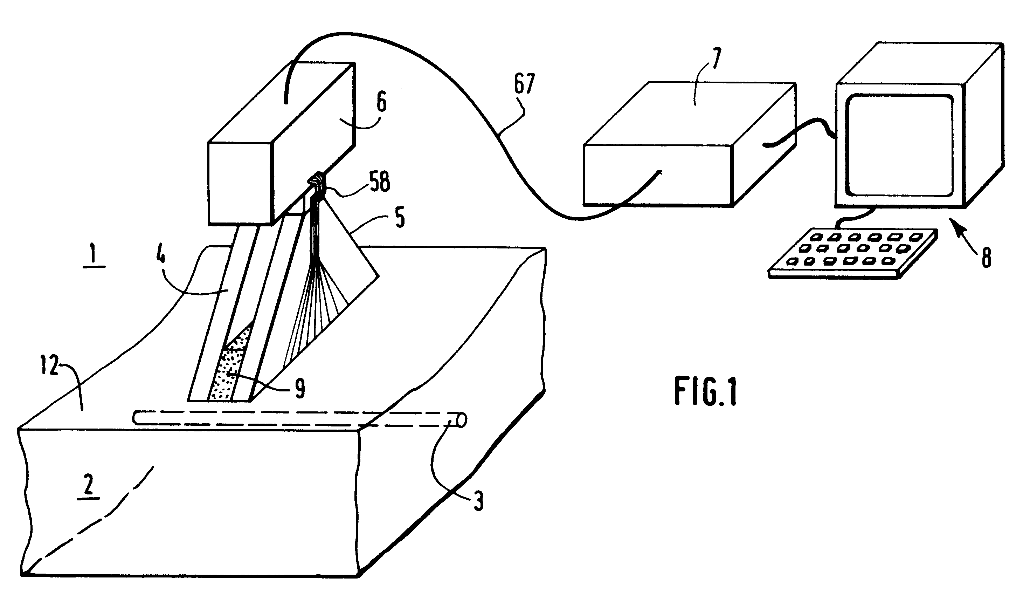

En se référant à la figure 1, un dispositif d'émission-réception est décrit, qui, disposé dans le milieu ambiant 1, permet de former une image d'une ou plusieurs armatures métalliques 3 noyées dans un bloc 2 de béton armé, afin notamment de déterminer la localisation précise et le diamètre de l'armature, par exemple, sans avoir à détruire une partie du béton 2.

Le dispositif comprend ici une tête de mesure comprenant principalement un circuit électronique micro-onde 6, une antenne d'émission 4 et une antenne de réception 5. Cette tête de mesure est reliée, par un cable souple 67, à un circuit électronique basse-fréquence 7, suivi d'une unité de traitement et de visualisation 8.

La tête de mesure est portable pour permettre l'exploration de l'ensemble de la surface de séparation 12, ou interface, entre le milieu ambiant 1 et le béton 2, si cela s'avère nécessaire, tandis que le circuit 7 et l'unité 8 restent au même endroit.

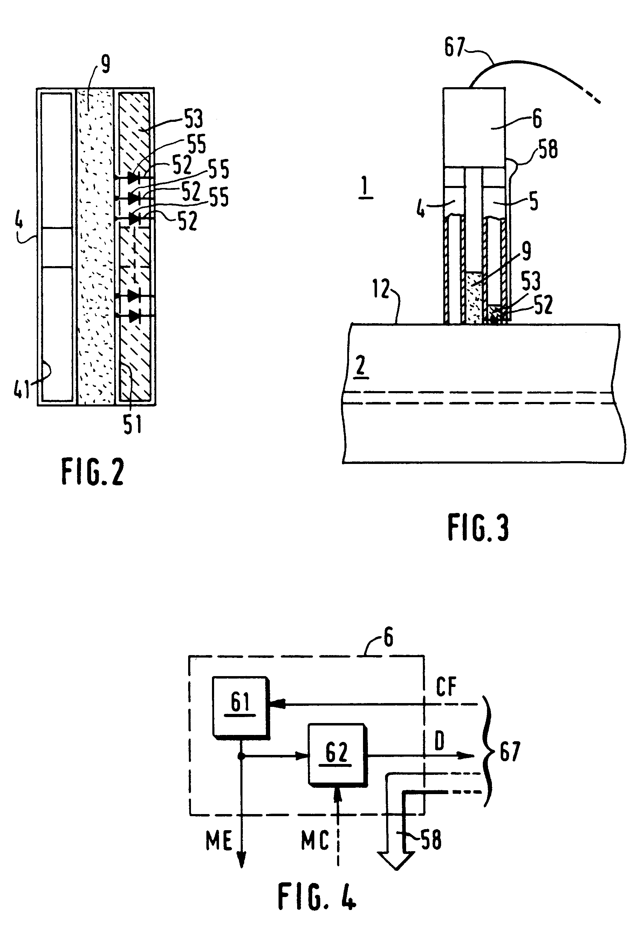

L'antenne d'émission est un cornet sectoral micro-onde, s'étendant dans le plan H, et qui, comme le montre la figure 2, est donc terminé par une ouverture rayonnante 41 sensiblement en forme de guide d'ondes rectangulaire.

En fonctionnement, l'ouverture 41 est appliquée contre la surface de séparation 12. La hauteur du cornet 4 et la longueur de l'ouverture 41 sont ici sensiblement égales à 20 λo, λo désignant la longueur d'onde du rayonnement micro-onde dans le milieu ambiant, en l'occurence de l'air, pour la fréquence centrale Fo de fonctionnement du dispositif. Ainsi, si la fréquence centrale est choisie, par exemple, égale à 10 GHz, la longueur d'onde λo vaut 3 cm, et la hauteur du cornet 4, ainsi que la longueur de l'ouverture 41 valent 60 cm. La largeur de l'ouverture 41, qui correspond à l'épaisseur du cornet 4, est ici de 1 cm environ.

L'antenne de réception 5 est un cornet identique au cornet 4, et qui comprend donc une ouverture collectrice 51 sensiblement en forme de guide d'ondes rectangulaire, comme le montre la figure 2. Comme cela apparaît sur cette figure, et encore plus clairement sur la figure 3, une lame diélectrique 53 est disposée dans l'ouverture collectrice 51 de l'antenne 5. L'épaisseur et les propriétés diélectriques de la lame 53 sont déterminées pour que l'antenne de réception soit adaptée au béton 2 lorsque, en fonctionnement normal, l'ouverture 51 est appliquée contre la surface de séparation 12. Une telle adaptation permet notamment au rayonnement en provenance du béton 2, et se dirigeant vers l'intérieur du cornet 5, de subir une reflexion aussi faible que possible lors du changement de milieu, et donc d'être collecté par le cornet 5 avec un bon niveau.

La détermination de l'épaisseur et des propriétés diélectriques de la lame 53, en fonction des propriétés de l'air et du béton est à la portée de l'homme du métier, en utilisant par exemple les méthodes décrites dans l'ouvrage "Microwave Filters, Impédance Matching Networks and Coupling Structures" de G.L. MATTHAEI et al., MAC GRAW HILL, (1964).

Ici, une pluralité d antennes ponctuelles 52, disposées sur une ligne droite, est noyée dans la lame 53 disposée dans l'ouverture collectrice 51. Chacune des antennes ponctuelles 52 est une antenne du type doublet électrique, chargée en son centre par une diode PIN. Les brins de chaque antenne doublet 52 sont parallèles à la direction du champ électrique dans le cornet 5, c'est-à-dire perpendiculaires au plan du cornet. Chaque antenne 52 a un brin relié à la masse, par exemple par soudure de l'extrémité de ce brin sur le bord de l'ouverture 51, et son autre brin est raccordé à un conducteur d'une nappe 58, faisant partie du câble souple 67.

Les antennes ponctuelles 52 sont ici disposées dans la partie centrale de l'ouverture 51, de façon à occuper au total une longueur égale à la moitié de celle de l'ouverture, ici de 10 λo, soit 30 cm. Les antennes 52 sont ici au nombre de 64, et donc leur pas d'alignement est donc voisin de λo/6, soit ici 0,5 cm.

Comme le montrent les figures 1, 2 et 3, l'ouverture collectrice 51 est disposée de façon telle qu'un de ses grands côtés et un des grands côtés de l'ouverture rayonnante 41 se trouvent côte à côte. Ici, une couche 9 de matériau absorbant le rayonnement micro-onde, dans la gamme des fréquences de travail, par exemple une mousse de matériau plastique chargée de carbone, est intercalée entre ces deux grands côtés des ouvertures 41 et 51. L'épaisseur de cette couche 9 est ici du même ordre de grandeur que l'épaisseur des cornets 4 et 5, et la hauteur de cette couche, ou plus exactement sa dimension perpendiculairement à la surface de séparation 12, est de l'ordre de λo, soit ici de 3 cm.

L'antenne d'émission 4 et l'antenne de réception 5 sont solidaires du circuit micro-onde 6, par montage sur un châssis commun. L'antenne d'émission 4 reçoit un signal micro-onde émis ME en provenance du circuit 6, et l'antenne de réception 5 délivre à ce circuit 6 un signal micro-onde collecté MC.

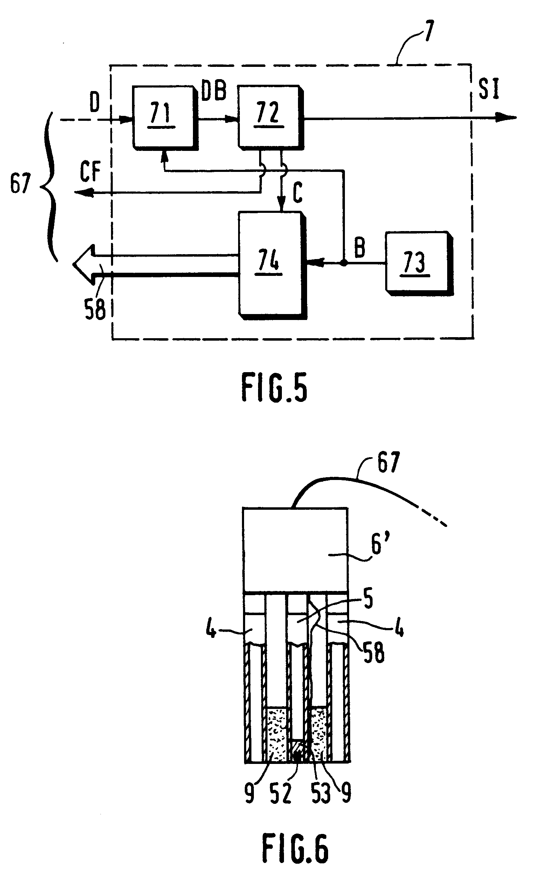

Comme le montre la figure 4, le circuit micro-onde 6 comprend une source micro-onde 61 et un circuit 62 de détection synchrone micro-onde. La source micro-onde 61 délivre le signal ME de fréquence F comprise entre 0,7 Fo et 1,3 Fo, ici 7 et 13 GHz respectivement. La fréquence du signal ME est commandée par un signal CF, par exemple un signal numérique, supporté par un ou plusieurs conducteurs faisant partie du câble 67. Le circuit 62, à large bande, reçoit le signal ME et le signal MC et délivre un signal D à un des conducteurs du câble 67.

Comme le montre la figure 5, le circuit basse-fréquence comprend un circuit 71 de détection synchrone basse-fréquence, un circuit 72 de commande et de traitement, un générateur basse-fréquence 73, et un multiplexeur 74.

Le générateur basse-fréquence 73 délivre un signal basse-fréquence B. Par "signal basse-fréquence", on entend ici un signal périodique, carré ou sinusoïdal, dont la gamme de fréquences est considérablement plus basse que la gamme des fréquences habituellement appelées micro-onde. Ainsi, comme on peut admettre que la gamme micro-onde est limitée vers le bas à quelques centaines de megahertz, le signal B aura en principe une fréquence inférieure à quelques dizaines de megahertz.

Le circuit 71 de détection synchrone basse fréquence est pourvu d'une entrée recevant le signal D, d'une entrée recevant le signal B, et d'une sortie délivrant un signal DB.

Le multiplexeur 74 est pourvu d'une unique entrée recevant le signal B, d'une pluralité de sorties reliées aux conducteurs de la nappe 58, et d'une entrée de commande recevant un signal numérique C.

Le circuit de traitement 72, géré à l'aide d'un micro-processeur, par exemple, est pourvu d'une entrée recevant le signal DB, et de trois sorties délivrant les signaux numériques CF, C et un signal SI également numérique représentatif du champ micro-onde mesuré.

L'unité de traitement et de visualisation 8 reçoit le signal SI, et exécute un logiciel de mise en oeuvre de la méthode, déjà citée, d'amélioration de la résolution spatiale de l'image, et visualise cette image sur l'écran dont il est pourvu.

Le dispositif qui vient d'être décrit fonctionne comme suit.

Le circuit de traitement 72 commande d'abord, par l'intermédiaire du signal CF, la source micro-onde pour que sa valeur soit, par exemple, de 7 GHz.

Le rayonnement micro-onde résultant est émis par le cornet 4, et se trouve en partie réfléchi par la surface de séparation 12, et en partie transmis dans le béton 2.

La partie réfléchie reste confinée à l'intérieur du cornet 4 d'émission, ou est absorbée par la couche 9, ce qui fait qu'une très faible partie seulement de ce rayonnement parasite atteint le cornet 5 de réception.

Par contre, la partie du rayonnement émis qui se trouve transmise dans le béton se réfléchit sur l'objet 3, et donne naissance à un rayonnement réfléchi collecté par le cornet 5.

Simultanément, le circuit de traitement 72 commande le multiplexeur pour que le signal B soit appliqué à une seule des diodes 55. Ainsi, seule cette diode est polarisée, successivement en direct et en inverse, au rythme du signal B.

Dans le circuit 62, le signal micro-onde collecté MC subit une détection synchrone micro-onde à l'aide du signal micro-onde ME, puis le signal résultant D subit, dans le circuit 71, une détection synchrone basse fréquence à l'aide du signal B. Or, seule la partie du rayonnement collecté en provenance de l'antenne doublet 52, chargée par la diode 55 polarisée par le signal B, se trouve modulée par ce signal B. De ce fait, le signal MC, après détection micro-onde, puis basse fréquence, n'est représentatif que du champ au point où se trouve l'antenne doublet 52 chargée par la diode 55 polarisée à l'aide du signal B. Le microprocesseur du circuit 72 peut donc commander la polarisation successive de chacune des diodes 55 et élaborer, à partir du signal C qui indique l'emplacement du point de mesure, et du signal résultant de la double détection qui indique la valeur du champ en ce point de mesure, le signal SI.

Ce type de fonctionnement résulte de la mise en oeuvre de la méthode connue par l'homme du métier sous le nom "méthode de diffusion modulée".

Après cette première mesure à 7 GHz, le circuit 72 commande une variation de la fréquence de la source 61, pour obtenir les mêmes résultats pour une autre valeur de la fréquence, et ainsi de suite, afin, par exemple, de permettre à l'unité 8 de mettre en oeuvre la méthode d'amélioration de la résolution déjà mentionnée. Ainsi, on arive à obtenir, dans chacune des deux directions d'un plan parallèle au plan des cornets 4 et 5, une résolution de l'ordre de la demi-longueur d onde minimale dans le béton, ce qui correspond, pour la gamme de fréquence 7-13 GHz, et en admettant pour le béton une constante diélectrique relative de 6, à une valeur de 0,5 cm environ, qui correspond évidemment au pas d'alignement des antennes doublets 52.

Sur la figure 6, on a représenté une variante particulièrement avantageuse de la tête de mesure, pour laquelle il est prévu deux antennes d'émission 4, identiques à celle qui vient d'être décrite, disposées symétriquement par rapport à une unique antenne de réception 5, identique à celle qui vient d'être décrite. Cette disposition symétrique permet l'élimination de certains signaux parasites et l'amélioration de la définition dans la direction perpendiculaire au plan des cornets 4 et 5. Le circuit micro-onde 6′ correspondant est adapté en conséquence.

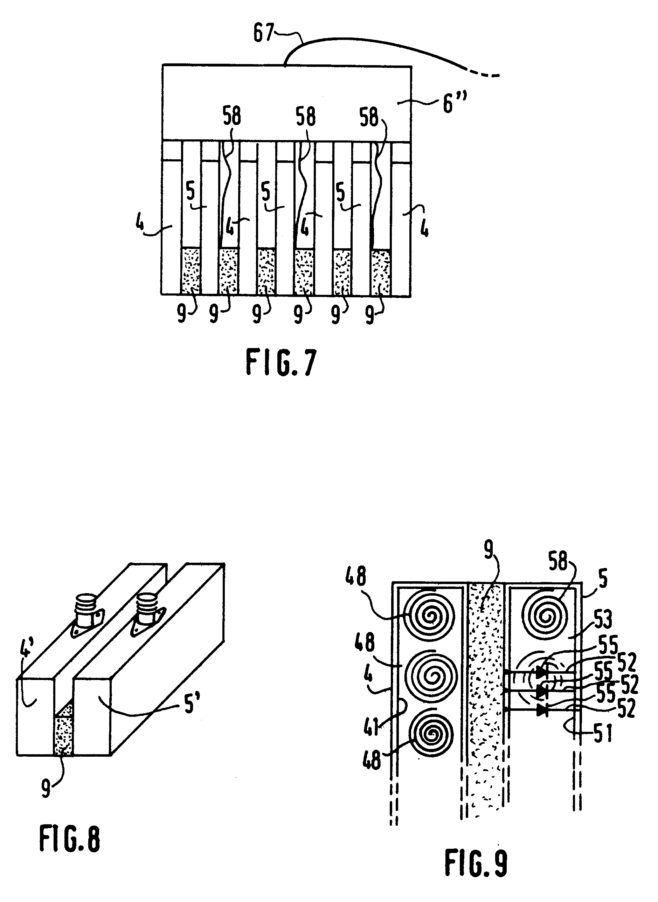

De même, sur la figure 7, on a représenté une autre variante de la tête de mesure, pour laquelle il est prévu une pluralité d'antennes d'émission 4, toutes identiques à celle qui vient d'être décrite, et une pluralité d'antennes de réception, toutes identiques à celle qui vient d'être décrite, juxtaposées de façon alternée. Le circuit micro-onde 6˝ correspondant est adapté en conséquence.

Sur les figures 8 et 9 sont représentées des variantes 4′ et 5′ des antennes d'émission et de réception 4 et 5. Il s'agit toujours, comme les cornets 4 et 5, d'antennes large-bande, mais elles sont réalisées chacune à l'aide d'un tronçon de guide d'ondes ouvert à une extrémité, et pourvu, à l'autre extrémité, d'un réseau d'antennes élémentaires large-bande, telle que des antennes en spirales 48 et 58, par exemple.

Comme pour les cornets 4 et 5, la grande dimension de la section des tronçons de guide d'ondes vaut ici 20 λo. Une telle valeur n'est toutefois pas impérative, il suffit que cette grande dimension vaille de une à quelques dizaines de λo. La petite dimension de la section des tronçons de guide est ici imposée par l'encombrement des antennes 48 et 58.

Naturellement, la portée de la présente demande n'est pas limitée à la description qui vient d'être faite.

Ainsi, on a décrit, dans un souci de simplification, l'amenée des signaux de polarisation basse-fréquence des diodes à l'aide d'une simple nappe de fils. En pratique, et comme l'homme de métier pourra le concevoir, il est intéressant d'amener ces signaux de polarisation par l'intermédiaire de filtres passe-bas, qui se comportent comme des courts-circuits pour les signaux basse-fréquence, et comme des circuits ouverts pour les signaux micro-ondes. Ces filtres peuvent avantageusement être réalisés à l'aide de lignes à rubans ("microstrip"), ou triplaques ("stripline") par exemple, disposées le long des parois des cornets 4 et 5 ou des tronçons des antennes 4′ et 5′.

De même, il n'est pas obligatoire d'utiliser des diodes PIN, et on peut les remplacer par des photodiodes polarisables par faisceau laser ou fibres optiques.

De même, il n'est pas obligatoire de mettre systématiquement en oeuvre l'algorithme d'amélioration de la résolution, et on peut également travailler à une seule fréquence en visualisant directement le signal SI.

Enfin, les applications du dispositif de l'invention ne sont évidemment pas limitées à l'auscultation non destructive du béton armé. Notamment, il est à la portée de l'homme de métier de transporter les longueurs d'onde utilisées en fonction de la taille, de la nature et de la profondeur des structures à détecter mais aussi des propriétés électromagnétiques du milieu dans lequel elles sont enfouies.

## Revendications

1. Dispositif d'émission et de réception d'un rayonnement, disposé dans un premier milieu (1) en vue de la formation, depuis ce premier milieu (1), d'une image d'un objet (3), noyé dans un deuxième milieu (2), comprenant :
- une source micro-onde (61),
- au moins une antenne d'émission (4 ; 4') raccordée à la source (61), et pourvue d'une ouverture rayonnante (41), sensiblement en forme de guide d'ondes rectangulaire, appliquée contre la surface (12) qui sépare le premier milieu (1) du deuxième milieu (2), et
- au moins une antenne de réception (5 ; 5') pourvue d'une ouverture collectrice (51) sensiblement en forme de guide d'ondes rectangulaire, appliquée contre la surface de séparation (12),
caractérisé par le fait que ladite antenne de réception est disposée pour qu'un grand côté de ladite ouverture rayonnante (41) et un grand côté de ladite ouverture collectrice (51) se trouvent côte à côte, ladite antenne de réception (5 ; 5') comprenant une ligne d'antennes ponctuelles (52) disposée dans ladite ouverture collectrice (51) et une sortie délivrant un signal micro-onde collecté (MC), et que le dispositif comprend :
- des moyens (73, 74) pour moduler successivement l'impédance de chacune desdites antennes ponctuelles (52) à l'aide d'un signal basse-fréquence (B), et,
- des moyens (62, 71, 72) pour déterminer, en réponse audit signal micro-onde collecté (MC) et audit signal basse-fréquence (B), le champ micro-onde à l'emplacement de chacune desdites antennes pontuelles (52).

2. Dispositif selon la revendication 1, dans lequel une couche (9) de matériau absorbant le rayonnement micro-onde est intercalée entre le grand côté de ladite ouverture rayonnante (41) et le grand côté de ladite ouverture collectrice (51) qui se trouvent côte à côte.

3. Dispositif selon l'une des revendications 1 ou 2, dans lequel lesdites antennes ponctuelles (52) sont noyées dans une lame diélectrique (53) agencée pour assurer l'adaptation de ladite antenne de réception (5 ; 5′) audit deuxième milieu (2).

4. Dispositif selon l'une des revendications 1 à 3, dans lequel il est prévu une unique antenne de réception (5) et deux antennes d'émission (4) disposées symétriquement par rapport à ladite antenne de réception (5).

5. Dispositif selon l'une des revendications 1 à 3, dans lequel il est prévu une pluralité d'antennes d'émission (4) et une pluralité d'antennes de réception (5), alternées.

6. Dispositif selon l'une des revendications 1 à 5, dans lequel :
- lesdits moyens de détermination du champ comprennent des moyens (62) de détection synchrone micro-onde dudit signal micro-onde collecté (MC) suivis de moyens (71,72) de détection et de traitement en basse fréquence,
- ladite source micro-onde (61), ladite antenne d'émission (4 ; 4′) ladite antenne de réception (5 ; 5′) et lesdits moyens (62) de détection synchrone micro-onde sont tous solidaires d'un premier châssis (6 ; 6′ ; 6˝) portable pour être déplacé en divers endroits de ladite surface de séparation (12), et,
- lesdits moyens (73, 74) de modulation et lesdits moyens (71, 72) de détection et de traitement en basse fréquence sont reliés audit premier châssis (6 ; 6′; 6˝) par au moins un câble souple (67).

7. Dispositif selon l'une des revendications 1 à 6, dans lequel lesdites antennes d'émission (4 ; 4′) et de réception (5 ; 5′) sont des antennes large - bande.

8. Dispositif selon la revendication 7, dans lequel les antennes d'émission et de réception sont des cornets (4, 5), et la grande dimension de ladite ouverture rayonnante (41) et de ladite ouverture collectrice (51) vaut de une à quelques dizaines de longueurs d'onde du rayonnement micro-onde dans ledit premier milieu (1).

9. Dispositif selon la revendication 7, dans lequel les antennes d'émission (4′) et de réception (5′) sont des tronçons de guide d'ondes, ouverts à une extrémité, et pourvus, à l'autre extrémité, d'un réseau d'antennes large-bande (48, 58), et la grande dimension de section dudit guide d'ondes vaut de une à quelques dizaines de longueurs d'onde du rayonnement micro-onde dans ledit premier milieu (1).

## Claims

1. A device for transmitting and receiving radiation, disposed in a first medium (1) for forming, from this first medium (1), an image of an object (3) buried in a second medium (2), comprising:
- a micro-wave source (61),
- at least one transmitting antenna (4 ; 4') connected to the source (61) and having a radiating opening (41) substantially in the form of a rectangular wave-guide, applied against the surface (12) which separates the first medium (1) from the second medium (2),
- at least one receiving antenna (5 ; 5') having a collecting opening (51) substantially in the form of a rectangular wave-guide applied against the separation surface (12), characterized in that said receiving antenna is disposed so that a large side of said radiating opening (11) and a large side of said collecting opening (51) are side by side, said receiving antenna (5 ; 5') comprising a line of pinpoint antennae (52) disposed in said collecting opening (51) and an output delivering a collected micro-wave signal (MC), and that the device comprises :
- means (73, 74) for successively modulating the impedance of each of said pinpoint antennae (52) by means of a low frequency signal (8), and
- means (62, 71, 72) for determining, in response to said collected micro-wave signal (MC) and said low frequency signal (B), the micro-wave field at the position of each of said pinpoint antennae (52).

2. The device according to claim 1, wherein a layer (9) of material absorbing the micro-wave radiation is inserted between the large side of said radiating opening (41) and the large side of said collecting opening (51) which are side by side.

3. The device according to one of claims 1 or 2, wherein said pinpoint antennae (52) are buried in a dielectric strip (53) adapted for matching said receiving antenna (5 ; 5') to said second medium (2).

4. The device according to one of claims 1 to 3, comprising a single receiving antenna (5) and two transmitting antennae (4), disposed symmetrically with respect to said receiving antenna (5).

5. The device according to one of claims 1 to 3, comprising a plurality of transmitting antennae (4) and a plurality of receiving antennae (5), which are alternated.

6. The device according to one of claims 1 to 5, wherein :
- said means for determining the field comprise means (62) for the micro-wave synchronous detection of said collected micro-wave signal (MC) followed by low frequency processing and detection means (71, 72),
- said micro-wave source (61), said transmitting antenna (4 ; 4') said receiving antenna (5 ; 5') and said micro-wave synchronous detection means (62) are all secured to a first chassis (6 ; 6' ; 6'') which is portable for being moved to different positions of said separation surface (12), and
- said modulation means (73, 74) and said low frequency processing and detection means (71, 72) are connected to said first chassis (6 ; 6' ; 6'') by at least one flexible cable (67).

7. The device according to one of claims 5 to 6, wherein said transmitting and receiving antennae (5 ; 5') are wide band antennae.

8. The device according to claim 7, wherein said transmitting and receiving antennae are horns (4, 5) and the large dimension of said radiating opening (41) and of said collecting opening (51) is from one to a few tens of wavelengths of the micro-wave radiation in said first medium (1).

9. The device as claimed in claim 7, wherein said transmitting (4') and receiving (5') antennae are wave-guide sections, open at one end, and having at the other end a network of wide band antennae (48, 58) and the large section dimension of said wave-guide is from one to a few tens of wavelengths of the micro-wave radiation in said first medium (1).

## Patentansprüche

1. Vorrichtung zum Aussenden und zum Empfang einer Strahlung, welche in einem ersten Medium (1) angeordnet ist, um von diesem ersten Medium (1) aus ein Bild eines Gegenstandes (3) zu erzeugen, der in einem zweiten Medium (2) eingebettet ist, umfassend:
- eine Mikrowellenquelle (61),
- mindestens eine Sendeantenne (4; 4'), die mit der Quelle (61) verbunden ist und mit einer strahlenden Öffnung (41) versehen ist, welche im wesentlichen die Form eines rechteckigen Wollenleiters besitzt und gegen die Fläche (12) gedrückt wird, die das erste Medium (1) von dem zweiten Medium (2) trennt, und
- mindestens eine Empfangsantenne (5; 5'), die mit einer Sammelöffnung (51) versehen ist, welche im wesentlichen die Form eines rechteckigen Wellenleiters besitzt und gegen die Trennfläche (12) gedrückt wird,
dadurch gekennzeichnet, daß die Empfangsantenne so angeordnet ist, daß eine lange Seite der strahlenden Öffnung (41) und eine lange Seite der Sammelöffnung (51) nebeneinanderliegen, wobei die Empfangsantenne (5; 5') in dieser Sammelöffnung (51) in einer Reihe angeordnete Punktantennen (52) sowie einen Ausgang umfaßt, der ein gesammeltes Mikrowellensignal (MC) liefert, und daß die Vorrichtung umfaßt:
- Mittel (73, 74), um nacheinander die Impedanz jeder der Punktantennen (52) mittels eines Niederfrequenzsignals (B) zu modulieren, und
- Mittel (62, 71, 72), um in Abhängigkeit von dem gesammelten Mikrowellensignal (MC) und dem Niederfrequenzsignal (B) das Mikrowellenfeld am Ort jeder der Punktantennen (52) zu bestimmen.

2. Vorrichtung nach Anspruch 1, bei der eine Schicht (9) aus einem Material, welches die Mikrowellenstrahlung absorbiert, zwischen der langen Seite der strahlenden Öffnung (41) und der langen Seite der Sammelöffnung (51), die sich nebeneinander befinden, angeordnet ist.

3. Vorrichtung nach einem der Ansprüche 1 oder 2, bei der die Punktantennen (52) in einer dielektrischen Schicht (53) eingebettet sind, die dafür vorgesehen ist, die Anpassung der Empfangsantenne (5; 5') an das zweite Medium (2) zu gewährleisten.

4. Vorrichtung nach einem der Ansprüche 1 bis 3, bei der eine einzige Empfangsantenne (5) und zwei in bezug auf die Empfangsantenne (5) symmetrisch angeordnete Sendeantennen (4) vorgesehen sind.

5. Vorrichtung nach einem der Ansprüche 1 bis 3, bei der eine Vielzahl von Sendeantennen (4) und eine Vielzahl von Empfangsantennen (5), die alternierend angeordnet sind, vorgesehen sind.

6. Vorrichtung nach einem der Ansprüche 1 bis 5, bei der:
- die Mittel zur Bestimmung des Felds Mittel (62) zur synchronen Mikrowellenerfassung des gesammelten Mikrowellensignals (MC), gefolgt von Mitteln (71, 72) zur Niederfrequenzerfassung und -verarbeitung, umfassen
- die Mikrowellenquelle (61), die Sendeantenne (4; 4'), die Empfangsantenne (5; 5') und die Mittel (62) zur synchronen Mikrowellenwellenerfassung sämtlich in einem ersten tragbaren Gehäuse (6; 6'; 6'') vereinigt sind, um an verschiedene Orte der Trennfläche (12) bewegt zu werden, und
- die Mittel (73, 74) zur Modulation und die Mittel (71, 72) zur Niederfrequenzerfassung und -verarbeitung mit dem ersten Gehäuse (6; 6'; 6'') durch mindestens ein Anschlußkabel (67) verbunden sind.

7. Vorrichtung nach einem der Ansprüche 1 bis 6, bei der die Sende- (4; 4') und die Empfangsantennen (5; 5') Breitbandantennen sind.

8. Vorrichtung nach Anspruch 7, bei der die Sande- und die Empfangsantennen Hörner (4, 5) sind und die große Abmessung der strahlenden Öffnung (41) und der Sammelöffnung (51) einem bis mehreren Zehn Wellenlängen der Mikrowellenstrahlung in dem ersten Medium (1) entspricht.

9. Vorrichtung nach Anspruch 7, bei der die Sende- (4') und die Empfangsantennen (5') Wellenleiterabschnitte sind, die an einem Ende geöffnet sind und an dem anderen Ende mit einem System von Breitbandantennen (48, 58) versehen sind, und die große Abmessung des Querschnitte dieses Wellenleiters einem bis mehreren Zehn Wellenlängen der Mikrowellenstrahlung in dem ersten Medium (1) entspricht.
